(19) Europäisches Patentamt
European Patent Office
Office européen des brevets

(11) Veröffentlichungsnummer: **0 152 598**
**B1**

# (12) EUROPÄISCHE PATENTSCHRIFT

(45) Veröffentlichungstag der Patentschrift: **20.04.88**

(51) Int. Cl.⁴: **C 07 D 243/08**, C 07 C 121/43, C 07 C 120/00 // A61K31/55

(21) Anmeldenummer: **84115471.9**

(22) Anmeldetag: **14.12.84**

(54) **Cyanomethyl-(2-cyano-ethyl)-(3-hydroxy-propyl)-amin, seine Verwendung zur Herstellung von 1-(3-Hydroxy-propyl)-1,4-diazepan und 1,4 Bis-[3-(3,4,5-trimethoxy-benzoyloxy)-propyl]-diazepan.**

(30) Priorität: **03.02.84 DE 3403778**

(43) Veröffentlichungstag der Anmeldung:
**28.08.85 Patentblatt 85/35**

(45) Bekanntmachung des Hinweises auf die Patenterteilung:
**20.04.88 Patentblatt 88/16**

(84) Benannte Vertragsstaaten:
**AT BE CH DE FR GB IT LI NL SE**

(56) Entgegenhaltungen:
**DE - B - 1 545 575**
**US - A - 1 972 465**
**US - A - 3 040 029**
**US - A - 3 980 643**

**JOURNAL OF THE AMERICAN CHEMICAL SOCIETY, Band 72, Nr. 4, 19. April 1950, Seiten 1814-1815; A.R. SURREY u.a.: "The preparation of 7-chloro-4-(4-(N-ethyl-N-beta-hydroxyethylamino)-1-methylbutylamino)-quinoline and related compounds"**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Bornweg 36, D-6052 Mühlheim 2 (DE)**
Erfinder: **Lehmann, Bernd, Dr., Ringstrasse 24a, D-7750 Konstanz 19 (DE)**
Erfinder: **Deller, Klaus, Dr., Friedhofstrasse 47, D-6452 Hainburg (DE)**

**EP 0 152 598 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die 1-(3-Hydroxy-propyl)-1,4-diazepan-gruppe ist Bestandteil verschiedener Arzneimittelwirkstoffe. Das 1-(3-Hydroxy-propyl)-1,4-diazepan dient daher auch als Ausgangsstoff für die Synthese von solchen Arzneimittelwirkstoffen (siehe zum Beispiel DE-OS 2 355 420). Das übliche Herstellungsverfahren für das 1-(3-Hydroxy-propyl)-1,4-diazepan besteht in der Umsetzung von 1,4-Diazepan (Homopiperazin) mit Allylalkohol oder 3-Chlor-propanol. Die Ausbeuten sind hierbei niedrig und betragen beispielsweise 34% beziehungsweise 46%. Ausserdem ist die Isolierung des Reaktionsproduktes umständlich und aufwendig. Der grösste Nachteil bei diesem Verfahren liegt jedoch darin, dass als Ausgangsstoff das 1,4-Diazepan verwendet wird. Zur Herstellung dieser Substanz gibt es zahlreiche Verfahren, die jedoch ebenfalls nur niedrige Ausbeuten liefern. Die zur Zeit beste Synthese besteht in der Umsetzung von Ethylendiamin mit Acrylnitril und anschliessender Hydrierung des erhaltenen N-(2-Cyano-ethyl)-ethylendiamins in Gegenwart von Raney-Nickel (siehe F. Poppelsdorf und R.C. Mayerly, J. Org. Chem., Band 26, 1961, Seite 131). Die Gesamtausbeute an 1-(3-Hydroxy-propyl)-1,4-diazepan bezogen auf N-(2-Cyano-ethyl)-ethylendiamin liegt jedoch auch bei diesem Verfahren nur bei 15%. Weiterhin muss bei diesem Verfahren die vierfach molare Menge Ethylendiamin pro 1 Mol Acrylnitril eingesetzt werden, um die Addition eines zweiten Moleküls Acrylnitril an das Reaktionsprodukt zurückzudrängen. Die Abtrennung des überschüssigen Ethylendiamins ist darüberhinaus schwierig und aufwendig.

Es wurde nun ein neuer verbesserter Weg zur Herstellung des 1-(3-Hydroxy-propyl)-1,4-diazepans gefunden, der erheblich einfacher in der Durchführung ist und es ausserdem erlaubt, diese Verbindung in erheblich besserer Ausbeute (53,5% bezogen auf Acrylnitril) als bisher zu erhalten. Dieser neue Weg basiert auf der neuen Verbindung Cyanomethyl-(2-cyano-ethyl)-(3-hydroxy-propyl)-amin, welche einerseits sehr einfach und in sehr hoher Ausbeute (beispielsweise 99% bezogen auf Acrylnitril) aus den leicht zugänglichen Ausgangsstofen 3-Aminopropanol, Acrylnitril, Formaldehyd und Blausäure in einem 1-stufigen Verfahren hergestellt werden kann und sich andererseits sehr glatt unter Ringschluss zum 1-(3-Hydroxy-propyl)-1,4-diazepan hydrieren lässt. Die sehr hohe Ausbeute von 87 bis 99% bezogen auf Acrylnitril bei der erfindungsgemässen Herstellung der neuen Verbindung Cyanomethyl-(2-cyano-ethyl)-(3-hydroxy-propyl)-amin ist überraschend.

Darüberhinaus ist auch der glatte Ringschluss des Cyanomethyl-(2-cyano-ethyl)-(3-hydroxy-propyl)-amins zum 1,4-Diazepan-Derivat bei der Hydrierung unerwartet. Beispielsweise ist bekannt, dass das unsubstituierte Cyanoethyl-cyanomethylamin unter üblichen Hydrierungsbedingungen nicht zum 1,4-Diazepan cyclisiert (siehe Acta Polon. Pharm. 18, 171–2 (1961); Acta Polon. Pharm. 19, 215–22 (1962), sondern dass nicht-cyclische Amine entstehen (als Hauptprodukt das N-(2-Amino-ethyl)-trimethylendiamin).

Weiterhin ist es im Gegensatz zu der bekannten Synthese des 1,4-Diazepans unter Verwendung von Ethylendiamin nach der erfindungsgemässen Ausführung nicht mehr notwendig, das 3-Amino-propanol im Überschuss einzusetzen.

Ausgehend von dem nunmehr leicht zugänglichen 1-(3-Hydroxy-propyl)-1,4-diazepan lässt sich 1,4-Bis-[3-(3,4,5-trimethoxy-benzoyloxy)-propyl]-diazepan nach bekannten Verfahren herstellen. Das 1,4-Bis-[3-(3,4,5-trimethoxy-benzoyloxy)-propyl]-diazepan ist ein bekannter Arzneimittelwirkstoff (DILAZEP).

Die Erfindung betrifft ein Verfahren zur Herstellung von 1,4-Bis-[3-(3,4,5-trimethoxy-benzoyloxy)-propyl]-diazepan der Formel

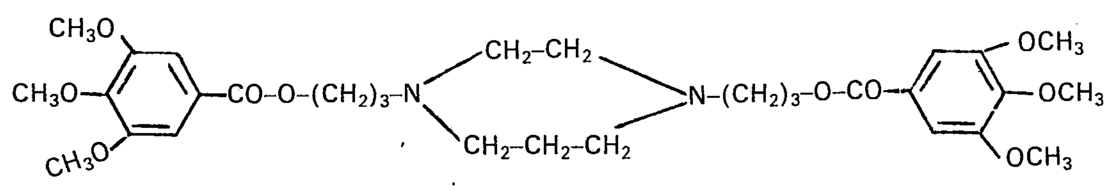

I

und dessen Salze in einer mehrstufigen Reaktion, wobei man in einer letzten Reaktionsstufe 1-(3-Hydroxy-propyl)-1,4-diazepan mit 3-Halogenpropanol oder mit Allylalkohol umsetzt und in das so erhaltene Reaktionsprodukt durch Veresterung der beiden Hydroxygruppen in an sich bekannter Weise zwei 3,4,5-Trimethoxy-benzoylreste einführt oder in das 1-(3-Hydroxy-propyl)-1,4-diazepan in wechselnder Reihenfolge durch Veresterung der Hydroxygruppe einen 3,4,5-Trimethoxy-benzoylrest und an das zweite freie Stickstoffatom des Diazepanringes die 3-[3,4,5-Trimethoxy -benzoyloxy]- propyl-(1)-gruppe einführt und gegebenenfalls das so erhaltene 1,4-Bis-[3-(3,4,5-trimethoxybenzoyloxy)-propyl]-diazepan der Formel I in ein Salz überführt, dadurch gekennzeichnet, dass man in einer ersten Reaktionsstufe

a) 3-Amino-propanol zuerst mit Acrylnitril in äquimolaren Mengen ohne Lösungsmittel bei Temperaturen zwischen 0 und 80 °C umsetzt und anschliessend das erhaltene

3-(2-Cyano-ethyl)-aminopropanol in wässriger Lösung mit Formaldehyd und Blausäure bei Temperaturen zwischen 0 und 80 °C bei einem pH oberhalb von 4 oder mit Formaldehyd und einem Alkalicyanid in Gegenwart von Alkalihydrogensulfit zum Cyanomethyl-(2-cyano-ethyl)-(3-hydroxy-propyl)-amin umsetzt und in einer zweiten Reaktionsstufe

b) das erhaltene Cyanomethyl-(2-cyano-ethyl)-(3-hydroxypropyl-amin in Gegenwart eines Hydrierungskatalysators und gegebenenfalls Ammoniak, mit Wasserstoff bei Temperaturen zwischen 20 bis 180 °C und Drucken zwischen 1 bis 300 bar zum 1-(3-Hydroxypropyl)-1,4-diazepan hydriert.

Die Umsetzung des 3-Amino-propanols mit Acrylnitril erfolgt unter Rühren beziehungsweise ständiger Durchmischung in äquimolaren Mengen ohne Lösungsmittel bei Temperaturen zwischen 0 bis 80 °C, vorzugsweise 20° und 40 °C.

Das so erhaltene (2-Cyano-ethyl)-(3-hydroxypropyl)-amin (Ausbeute 99,5% der Theorie bezogen auf Acrylnitril) kann ohne zusätzliche Reinigung direkt für die weitere Cyanomethylierung verwendet werden.

Selbstverständlich kann das aus 3-Amino-propanol und Acrylnitril primär erhaltene (2-Cyanoethyl)-(3-hydroxypropyl)-amin auch isoliert werden und dann erst mit Formaldehyd und einem Alkalisalz der Blausäure in Gegenwart eines Alkalihydrogensulfits oder ohne Alkalisulfitzusatz mit Formaldehyd und Blausäure umgesetzt werden.

Cyanomethylierung des (2-Cyano-ethyl)-(3-hydroxy-propyl)-amins:

Diese Reaktion erfolgt unter Rühren beziehungsweise ständiger Durchmischung der Reaktionsteilnehmer in wässrigem Medium bei Temperaturen zwischen 0 und 80, vorzugsweise 20 und 50 °C. Im Falle der Verwendung von Formaldehyd/Blausäure erfolgt die Reaktion bei einem pH oberhalb von 4, vorzugsweise 6. Es kann wasserfreie oder wasserhaltige Blausäure verwendet werden. Die Einstellung des pH-Wertes erfolgt, falls notwendig, beispielsweise mittels Natronlauge.

Falls eine stabilisierte Blausäure verwendet wird, kann gegebenenfalls ein entsprechender Zusatz von Alkalilauge (NaOH) erforderlich werden, um den Stabilisator zu neutralisieren.

Pro 1 Mol eingesetztes 3-Amino-propanol werden 1,0 bis 1,1 Mol Formaldehyd und 1,0 bis 1,3 Mol Blausäure verwendet. Das (2-Cyanoethyl)-(3-hydroxy-propyl)-amin wird zweckmässig bei einer Temperatur zwischen 0 und 80 °C in eine wässrige Lösung zugegeben, die Formaldehyd enthält. Die Mischung wird 60 bis 10 Minuten bei 20 bis 80 °C gehalten und dann während 120 bis 10 Minuten Blausäure zugegeben.

Bei Verwendung von Formaldehyd und einem Alkalisalz (Na-K-Salz) der Blausäure arbeitet man in Anwesenheit eines Alkalihydrogensulfits (Na-K-Salz).

Anstelle von Formaldehyd können auch andere formaldehydliefernde Substanzen verwendet werden, beispielsweise Paraformaldehyd. Bei Verwendung von Paraformaldehyd wird das Verfahren wie folgt abgeändert: Das Reaktionsgemisch wird bis zur vollständigen Auflösung des Paraformaldehyds gerührt.

Cyclisierung des Cyanomethyl-(2-cyanoethyl)-(3-hydroxy-propyl)-amins zum 1-(3-Hydroxy-propyl)-1,4-diazepan (N-(3-Hydroxypropyl)-homopiperazin):

Diese Hydrierungsreaktion wird mit Wasserstoff bei Temperaturen zwischen 20–180 °C, vorzugsweise 50–140 °C, insbesondere 70–120 °C und Drucken zwischen 1–300 bar, vorzugsweise 50–150 bar, insbesondere 70–110 bar vorgenommen.

Als Hydrierungs-Katalysatoren kommen in Frage: Edelmetallkatalysatoren (beispielsweise Pt, Pd, Rh, Ru, $PtO_2$ beziehungsweise Gemische dieser Katalysatoren) in freier Form oder als Trägerkatalysatoren (zum Beispiel auf Aktiv-Kohle, $BaSO_4$, $Al_2O_3$) oder Nichtedelmetallkatalysatoren, insbesondere Ni- oder Co-Katalysatoren. Die Nichtedelmetallkatalysatoren können ebenfalls metallisch, auf Trägern (zum Beispiel auf $SiO_2$, Kieselgur, $Al_2O_3$) oder insbesondere in aktivierter Form (zum Beispiel des Typs Raney) angewendet werden. Die Katalysatormenge kann zum Beispiel 1–80%, vorzugsweise 2–40%, insbesondere 10–30% der eingesetzten Cyanomethyl-(2-cyano-ethyl)-(3-hydroxy-propyl)-amin-Menge betragen.

Als Lösungsmittel für die Hydrierungs-Reaktion kommen zum Beispiel in Betracht: Wasser, symmetrische oder unsymmetrische Alkylether mit Alkylgruppen von 1–6 C-Atomen, gesättigte cycloaliphatische Ether wie Tetrahydrofuran, Dioxan, $C_1$–$C_6$-Alkanole (Methanol, Ethanol, Isopropanol), vorzugsweise $C_1$–$C_5$-Alkanole. Diese Mittel können auch als Gemische verwendet werden. Die Konzentration des Cyanomethyl-(2-cyano-ethyl)-(3-hydroxy-propyl)-amins in dem Lösungsmittel kann zum Beispiel zwischen 0,1–70%, vorzugsweise 1–40% sein. Die Hydrierung erfolgt in Gegenwart von Ammoniak. Pro 1 Mol Cyanomethyl-(2-cyano-ethyl)-(3-hydroxypropyl)-amin werden beispielsweise 0–50 Mol $NH_3$, vorzugsweise 0,1–30 Mol $NH_3$, insbesondere 1–15 Mol $NH_3$ angewendet.

Für die Hydrierung ist das Cyanomethyl-(2-cyano-ethyl)-(3-hydroxy-propyl)-amin insbesondere auch als Rohprodukt (das heisst beispielsweise verunreinigt durch Acrylnitril) geeignet. In einem solchen Fall ist es dann zweckmässig, einen hochaktiven Katalysator, wie zum Beispiel einen frisch hergestellten Katalysator (zum Beispiel frisch hergestelltes Raney-Nickel) zu verwenden.

Setzt man andererseits besonders reines, das heisst umkristallisiertes Cyanomethyl-(2-cyanoethyl)-(3-hydroxy-propyl)-amin ein, dann empfiehlt sich häufig in diesem Fall für die Hydrierung einen weniger aktiven Katalysator, wie zum Beispiel handelsübliches Raney-Nickel, einzusetzen.

Die Umsetzung des 1-(3-Hydroxy-propyl)-1,4-diazepans mit 3-Halogenpropanol (Halogen vorzugsweise Chlor oder Brom) kann ohne Lösungsmittel oder in Gegenwart von gegenüber dem 1-(3-Hydroxy-propyl)-1,4-diazepan und 3-Chlorpropanol weitgehend inerten Lösungsmitteln durchgeführt werden. Arbeitet man unter Normaldruck, so muss das verwendete Lösungsmittel beziehungsweise Lösungsmittelgemisch einen Siedepunkt haben, der mindestens der Reaktionstemperatur entspricht. Als Lösungsmittel kommen zum Beispiel in Frage: niedere aliphatische Alkohole (Methanol, Ethanol, Propanol, Isopropanol, n-Butanol), aromatische Kohlenwasserstoffe, wie Toluol, Xylol, cyclische Äther wie Dioxan, gesättigte aliphatische Ether wie Ethylenglykoldimethylether. Die Reaktionstemperatur kann zwischen 60 und 170 °C, vorzugsweise zwischen 90 und 130 °C liegen. Die Reaktionszeit sinkt mit steigender Temperatur.

Die Umsetzung des 1-(3-Hydroxy-propyl)-1,4-diazepans mit 3-Chlorpropanol kann in Abwesenheit oder in Anwesenheit einer Hilfsbase durchgeführt werden. In Abwesenheit einer Base erhält man das 1,4-Bis-(3-hydroxy-propyl)-1,4-diazepan als Monohydrochlorid. Als Hilfsbasen können organische und anorganische Basen verwendet werden. Vorzugsweise verwendet man Basen, deren Chloride im Reaktionsgemisch unlöslich sind und durch Filtration abgetrennt werden können, wie zum Beispiel Kaliumcarbonat, Natriumcarbonat, Natriummethylat.

Pro Mol 1-(3-Hydroxy-propyl)-1,4-diazepan werden 1 bis 1,5 Mol 3-Chlorpropanol und 0 bis 1,5 Mol Hilfsbase eingesetzt.

Anstelle des 1-(3-Hydroxy-propyl)-1,4-diazepans kann auch ein Salz desselben, insbesondere das Dihydrochlorid verwendet werden. In diesem Fall ist der Zusatz einer mindestens äquivalenten Menge an Hilfsbase notwendig.

Die Umsetzung des 1-(3-Hydroxy-propyl)-1,4-diazepans mit Allylalkohol kann mit oder ohne Lösungsmittel durchgeführt werden. Vorzugsweise wird jedoch, ausser Allylalkohol selbst, kein Lösungsmittel zugesetzt. Die Reaktion wird durch starke Basen, insbesondere Alkalialkoholate wie Natriumallylalkohol, Alkalioxide und Alkalihydroxide katalysiert.

Die Reaktionstemperatur kann zwischen 80 und 190 °C, vorzugsweise zwischen 95 und 160 °C liegen. Bei Reaktionstemperaturen oberhalb des Siedepunktes von Allylalkohol arbeitet man unter Druck bis zu 13 bar. Die Reaktionszeit sinkt mit steigender Temperatur.

Veresterung beziehungsweise Acylierung des 1,4-Bis-(3-hydroxy-propyl)-1,4-diazepans:

Die Acylierung kann beispielsweise in inerten Lösungs- beziehungsweise Suspensionsmitteln wie aliphatischen $C_3$–$C_9$-Ketonen, Dioxan, Dimethylformamid, Benzol, Toluol bei Temperaturen zwischen 0 bis 200 °C, vorzugsweise 20 bis 150 °C, erfolgen. Als Acylierungsmittel kommen in Betracht: Säurehalogenide (Chloride, Bromide, Jodide), Säureanhydride oder Säureester der 3,4,5-Trimethoxybenzoesäure oder die freie Säure selbst; gegebenenfalls unter Zusatz eines säurebindenden Mittels wie eines tertiären Amins, zum Beispiel Triäthylamin oder Pyridin.

Pyridin kann gleichzeitig auch als Lösungsmittel verwendet werden. Bei den Estern handelt es sich insbesondere um solche mit niederen aliphatischen Alkoholen. Bei der Acylierung kann man auch so vorgehen, dass man erst von der umzusetzenden Verbindung eine Alkaliverbindung herstellt, indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) bei Temperaturen zwischen 0 und 150 °C umsetzt und dann das acylierende Agens zufügt.

Falls die freie Säure verwendet wird, so ist deren Aktivierung durch die Gegenwart von Kondensationsmitteln wie Dicyclohexylcarbodiimid, Schwefligsäure-bis-alkylamiden (zum Beispiel $SO[N(CH_3)_2]_2$, N,N'Carbonyldiimidazol und so weiter) erforderlich (Organic Reactions, Vol. 12, 1962, Seiten 205 und 239).

In einer anderen Ausführung kann die Hydroxygruppe des 1-(3-Hydroxy-propyl)-1,4-diazepans zuerst mit der 3,4,5-Trimethoxy-benzoesäure verestert werden und anschliessend die zweite freie NH-Gruppe des 1,4-Diazepan-Ringes durch die 3-[3,4,5-Trimethoxy-benzoyloxy]propylgruppe alkyliert werden. Diese beiden Reaktionen können natürlich auch in umgekehrter Reihenfolge durchgeführt werden.

Die Veresterung mit der 3,4,5-Trimethoxy-benzoesäure verläuft vorteilhafterweise in Gegenwart eines Entwässerungsmittels, wie zum Beispiel Chlorwasserstoffsäure, Schwefelsäure, p-Toluolsulfonsäure, Dicyclohexylcarbodiimid, Schwefligsäure-bis-alkylamiden, N,N'-Carbonyldiimidazol usw., wobei die Verwendung von p-Toluolsulfonsäure besonders bevorzugt wird. Bei der Veresterung können beliebige Lösungsmittel verwendet werden, sofern sie die Reaktion nicht verhindern. Typische Beispiele für Lösungsmittel sind: Benzol, Toluol, Xylol, Dioxan, Chloroform, Tetrahydrofuran usw., wobei Toluol oder Dioxan bevorzugt werden. Die Reaktionstemperatur liegt im allgemeinen vorteilhafterweise über Raumtemperatur, besonders bevorzugt nahe dem Siedepunkt der verwendeten Lösungsmittel.

Die anschliessende Einführung der 3-[3,4,5-Trimethoxybenzoyloxypropylgruppe erfolgt beispielsweise durch Umsetzung mit einem 3-[3,4,5-Trimethoxy- benzoyloxy]- propylhalogenid (insbesondere dem Chlorid oder Bromid) in einem inerten Lösungs- oder Suspensionsmittel bei Temperaturen zwischen 50 und 180 °C, vorzugsweise 70 bis 140 °C. Als Lösungsmittel und basische Hilfsstoffe kommen die gleichen in Frage, die bereits für die Umsetzung des 1-(3-Hydroxy-propyl)-1,4-diazepans mit 3-Halogenpropanol angegeben wurden. Auch die sonstigen Bedingungen sind analog den dort genannten Bedingungen anzuwenden.

Falls die Einführung der 3-[3,4,5-Trimethoxybenzoyloxy]-propylgruppe zuerst erfolgt, kann die anschliessende Veresterung ausserdem zum Beispiel auch durch Umsetzung mit einem 3,4,5-Trimethoxy-benzoylhalogenid (vorzugsweise Chlorid oder Bromid) oder einem Anhydrid oder Säureester des 3,4,5-Trimethoxy-benzoylhalogenids unter den bereits angegebenen Bedingungen erfolgen.

Beispiel 1

Synthese von Cyanomethyl-(2-cyano-ethyl)-(3-hydroxypropyl)-amin:

600,8 g (8,0 Mol) 3-Amino-propanol werden

innerhalb von 75 Minuten bei 25 °C mit 424,5 g (8,0 Mol) Acrylnitril versetzt. Man lässt 1 Stunde bei 25 °C nachreagieren und tropft dann das erhaltene 3-(2-Cyanoethyl)-aminopropanol innerhalb von 60 Minuten bei 20 bis 40 °C zu 840 g (8,4 Mol) 30%iger wässriger Formaldehydlösung. Im Anschluss an eine 30minütige Nachreaktion bei 40 °C werden bei der gleichen Temperatur innerhalb von 45 Minuten 239 g (8,84 Mol) wasserfreie Blausäure zugetropft. Man lässt 2 Stunden bei 40 °C nachreagieren. Es wird abgekühlt, mit 800 ml Methylenchlorid versetzt und die gebildete untere organische Phase abgetrennt. Die wässrige Phase wird noch einmal mit 250 ml Methylenchlorid extrahiert. Nach dem Einengen der vereinigten organischen Phasen verbleiben 1325 g (99% der Theorie bezogen auf Acrylnitril) eines farblosen Öls, das bei Raumtemperatur kristallisiert.

F.: 28–33 °C.

Beispiel 2

Synthese von Cyanomethyl-(2-cyano-ethyl)-(3-hydroxypropyl)-amin:

Zu 100 g (1 Mol) 30%iger Formaldehydlösung gibt man innerhalb von 15 Minuten bei 30 °C eine Lösung von 109,5 g (1 Mol) 95%igem Natriumhydrogensulfit in 190 ml Wasser. Anschliessend werden 128 g 3-(2-Cyanoethyl)aminopropanol bei 40 °C innerhalb von 30 Minuten zudosiert. Man lässt 15 Minuten nachreagieren und versetzt dann innerhalb von 35 Minuten bei 40 °C mit einer Lösung von 78 g (1,15 Mol) 96%igem Kaliumcyanid in 150 ml Wasser. Im Anschluss an eine einstündige Nachreaktion bei 40 °C wird auf 25 °C abgekühlt und die obere organische Phase abgetrennt. Die in 200 ml Methylenchlorid aufgenommene organische Phase wird mit 2 g Aktivkohle, 2 g Kieselgur und 14 g wasserfreiem Natriumsulfat gerührt. Nach Filtration wird das Lösungsmittel abdestilliert, wobei 146 g (0,873 Mol; 87% der Theorie bezogen auf Acrylnitril) Cyanomethyl-(2-cyano-ethyl)- (3-hydroxy-propyl)-amin als gelbliches Öl anfallen, das bei 22 °C kristallisiert. Die Umkristallisation aus Essigester liefert ein analysenreines Produkt vom Schmelzpunkt 36 °C:

IR (Film) Banden bei: 3440, 2940, 2840, 2240, 1135, 1060 $cm^{-1}$.

Beispiel 3

Synthese von (2-Cyano-ethyl)-(3-hydroxy-propyl)-amin:

75 g (1 Mol) 3-Amino-propanol werden innerhalb einer Stunde bei 25 °C mit 53 g (1 Mol) Acrylnitril versetzt. Man lässt 1 Stunde bei 40 °C nachreagieren. Das so erhaltene Reaktionsprodukt (128 g) wird direkt weiter mit Formaldehyd/Blausäure beziehungsweise Formaldehyd/Alkalisalz der Blausäure in Gegenwart eines Alkalihydrogensulfits umgesetzt.

Beispiel 4

Synthese von 1-(3-Hydroxypropyl)-1,4-diazepan:

a) 146 g (0,873 Mol) Cyanomethyl-(2-cyano-ethyl)-(3-hydroxy-propyl)-amin werden in einer Mischung aus 1460 ml Methanol und 365 g Ammoniak gelöst. Zusammen mit 87 g Raney-Nickel wird die Lösung in einem Autoklaven bei 80 bar Wasserstoffdruck und 80 °C bis zum Ende der Wasserstoffaufnahme hydriert (5 Stunden).

Die vom Katalysator abgetrennte Lösung wird eingedampft, wobei Methanol und Ammoniak zurückgewonnen werden.

Der Eindampfrückstand wird im Vakuum rektifiziert. Als Hauptlauf erhält man 75 g (54% der Theorie) 1-(3-Hydroxypropyl)- 1,4-diazepan vom Siedepunkt 97 °C bei 0,13 mbar. Der Berechnungsindex beträgt $n_D^{20} = 1,5000$.

b) 100 g (0,598 Mol) Cyanomethyl-(2-cyano-ethyl)-(3-hydroxy-propyl)-amin, 400 ml Isopropanol, 56 g Ammoniak und 24 g Raney-Nikkel werden in einem Autoklaven während 10 Stunden unter 100 bar Wasserstoffdruck auf 100 °C erhitzt.

Die vom Katalysator abgetrennte Lösung wird eingedampft und der Rückstand im Vakuum rektifiziert. Als Hauptlauf erhält man 51 g (54% der Theorie) Endprodukt.

c) 100 g (0,598 Mol) Cyanomethyl-(2-cyano-ethyl)-(3-hydroxy-propyl)-amin, 800 ml Ethanol, 67 g Ammoniak und 26 g Raney-Nickel werden in einem Autoklaven während 2 Stunden unter 80 bis 100 bar Wasserstoffdruck auf 70 °C (am Anfang) bis 120 °C (gegen Ende) erhitzt. Nachdem das Reaktionsgemisch wie unter b) beschrieben aufgearbeitet wurde, erhält man 52 g (55% der Theorie) Endprodukt.

Beispiel 5

79 g (0,5 Mol) 1-(3-Hydroxy-propyl)-1,4-diazepan werden in 200 ml n-Butanol gelöst und mit 52,0 g (0,55 Mol) 3-Chlorpropanol versetzt. Man erhitzt 4 Stunden unter Rückfluss zum Sieden, versetzt dann mit 99 g (0,55 Mol) 30%iger Natriummethylatlösung und destilliert anschliessend Methanol über eine Kolonne ab. Das Reaktionsgemisch wird abgekühlt und filtriert. Der aus Kochsalz bestehende Filterkuchen wird mit 80 ml n-Butanol nachgewaschen. Das Filtrat wird eingeengt, wobei n-Butanol rückgewonnen wird.

Als Rückstand verbleiben 108 g (0,5 Mol) öliges 1,4-Bis-(3-hydroxy-propyl)-1,4-diazepan. Zur weiteren Reinigung kann dieses in einem Gemisch aus 720 ml Ethanol und 100 ml Methanol aufgenommen und mit 38 g Chlorwasserstoffgas auf pH 0,5 gestellt werden. Beim Abkühlen kristallisieren 116 g (80% der Theorie) reines 1,4-Bis- (3-Hydroxy-propyl)- 1,4-diazepan-dihydrochlorid aus.

Beispiel 6

11,5 g (0,5 Mol) Natrium werden unter Rückflusskühlung in 310 g Allylalkohol gelöst. Nach Zugabe von 79 g (0,5 Mol) 1-(3-Hydroxy-propyl)-1,4-diazepan erhitzt man 80 Stunden unter Rückfluss. Man kühlt ab und leitet unter Rühren und Kühlen 18 g (0,5 Mol) HCl-Gas ein. Das ausgeschiedene Natriumchlorid wird abfiltriert

und mit 50 ml Allylalkohol nachgewaschen. Das Filtrat wird eingeengt, es verbleiben 101 g (0,47 Mol) öliges 1,4-Bis-(3-hydroxy-propyl)-1,4-diazepan. Zur weiteren Reinigung kann dieses in einem Gemisch aus 720 ml Ethanol und 100 ml Methanol aufgenommen und mit 38 g HCl-Gas auf pH 0,5 gestellt werden.

Beim Abkühlen kristallisieren 77 g (53% der Theorie) 1,4-Bis-(3-hydroxy-propyl)-1,4-diazepan-dihydrochlorid aus.

Beispiel 7

1,4-Bis-[(3,4,5-trimethoxy-benzoyloxy)-propyl]-diazepan

21,6 g 1,4-Bis-(3-hydroxy-propyl)-1,4-diazepan und 63,8 g 3,4,5-Trimethoxybenzoylchlorid werden in 600 Volumenteilen trockenem Chloroform gelöst und 5 Stunden zum Sieden erhitzt. Das Chloroform wird anschliessend im Vakuum abgedampft. Der Rückstand wird in Wasser aufgenommen und mit Äther gewaschen. Der wässrige Anteil wird alkalisch gemacht, die ölig anfallende Base in Äther aufgenommen und über $Na_2SO_4$ getrocknet. Nach dem Abdampfen des Äthers im Vakuum wird der sehr viskose Rückstand in 150 Volumenteilen Äthylenalkohol gelöst und mit der berechneten Menge HCl-Äther versetzt. Das alsbald auskristallisierende Dihydrochlorid wird abgesaugt, getrocknet und aus 120 Volumenteilen Äthanol umkristallisiert. Man erhält nach 3tägigem Trocknen über $P_2O_5$ 45 bis 50 g (55 bis 70% der Theorie) 1,4-Bis-[(3,4,5-trimethoxy-benzoyloxy)-propyl]-diazepan ·2 HCl · $H_2O$ mit dem Schmelzpunkt 194 bis 198 °C.

## Patentansprüche

1. Verfahren zur Herstellung von 1,4-Bis-[3-(3-(3,4,5-trimethoxy-benzoyloxy)- propyl]-diazepan der Formel

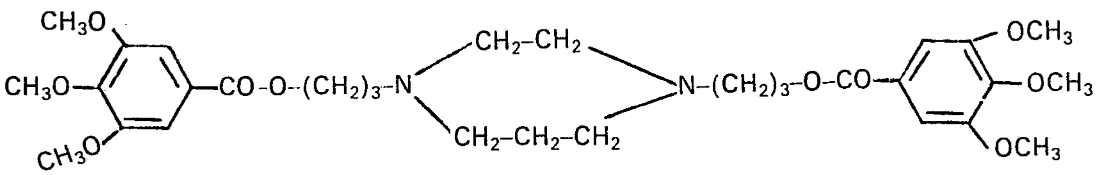

I

und dessen Salze in einer mehrstufigen Reaktion, wobei man in einer letzten Reaktionsstufe 1-(3-Hydroxy-propyl)-1,4-diazepan mit 3-Halogenpropanol oder mit Allylalkohol umsetzt und in das so erhaltene Reaktionsprodukt durch Veresterung der beiden Hydroxygruppen in an sich bekannter Weise zwei 3,4,5-Trimethoxy-benzoylreste einführt oder in das 1-(3-Hydroxy-propyl)-1,4-diazepan in wechselnder Reihenfolge durch Veresterung der Hydroxygruppe einen 3,4,5-Trimethoxy-benzoylrest und an das zweite freie Stickstoffatom des Diazepanringes die 3-[3,4,5-Trimethoxy-benzoyloxy]-propyl-(1)-gruppe einführt und gegebenenfalls das so erhaltene 1,4-Bis- [3-(3,4,5-trimethoxybenzoyloxy)-propyl]-diazepan der Formel I in ein Salz überführt, dadurch gekennzeichnet, dass man in einer ersten Reaktionsstufe

a) 3-Amino-propanol zuerst mit Acrylnitril in äquimolaren Mengen ohne Lösungsmittel bei Temperaturen zwischen 0 und 80 °C umsetzt und anschliessend das erhaltene 3-(2-Cyano-ethyl)-aminopropanol in wässriger Lösung mit Formaldehyd und Blausäure bei Temperaturen zwischen 0 und 80 °C bei einem pH oberhalb von 4 oder mit Formaldehyd und einem Alkalicyanid in Gegenwart von Alkalihydrogensulfit zum Cyanomethyl-(2-cyanoethyl)-(3-hydroxy-propyl)-amin umsetzt und in einer zweiten Reaktionsstufe

b) das erhaltene Cyanomethyl-(2-cyanoethyl)-(3-hydroxypropyl)-amin in Gegenwart eines Hydrierungskatalysators und gegebenenfalls Ammoniak, mit Wasserstoff bei Temperaturen zwischen 20 bis 180 °C und Drucken zwischen 1 bis 300 bar zum 1-(3-Hydroxypropyl)-1,4-diazepan hydriert.

2. Cyanomethyl-(2-cyano-ethyl)-(3-hydroxypropyl)-amin der Formel

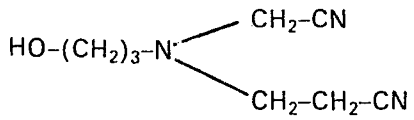

II.

3. Verfahren zur Herstellung von Cyanomethyl-(2-cyanoethyl)-(3-hydroxy-propyl)-amin der Formel II, dadurch gekennzeichnet, dass 3-(2-Cyano-ethyl)-aminopropanol in wässriger Lösung mit Formaldehyd und Blausäure bei Temperaturen zwischen 0 und 80 °C bei einem pH oberhalb von 4 oder in Gegenwart von Alkalihydrogensulfit mit Formaldehyd und einem Alkalisalz der Blausäure umgesetzt wird.

4. Verfahren nach Anspruch 3, dadurch gekennzeichnet, dass das (2-Cyano-ethyl)-(3-hydroxy-propyl)-amin in Form der durch Umsetzung von 3-Amino-propanol und Acrylnitril erhaltenen Reaktionsmischung eingesetzt wird.

5. Verwendung von Cyanomethyl-(2-cyanoethyl)-(3-hydroxy-propyl)-amin zur Herstellung von 1-(3-hydroxy-propyl)-1,4-diazepan.

6. Verwendung von Cyanomethyl-(2-cyanoethyl)-(3-hydroxy-propyl)-amin gemäss Anspruch 5, dadurch gekennzeichnet, dass Cyanomethyl- (2-cyano-ethyl)- 3-hydroxy-propyl)-amin in Gegenwart eines Hydrierungskatalysators hydriert wird.

7. Verfahren zur Herstellung von 1-(3-Hydroxyl-propyl)-1,4-diazepan durch hydrierende Cyclisierung von Cyanomethyl-(2-cyano-ethyl)-3-hydroxy-propyl)-amin in Gegenwart eines Hydrierungskatalysators und gegebenenfalls Ammoniak, mit Wasserstoff bei Temperaturen zwischen 20 bis 180 °C und Drucken zwischen 1 bis 300 bar.

8. Verwendung von Cyanomethyl-(2-cyanoethyl)-(3-hydroxy-propyl)-amin zur Herstellung

von 1,4-Bis-[3-(3,4,5-trimethoxy-benzoyloxy)-propyl]-diazepan.

## Revendications

1. Procédé pour la préparation de 1,4-bis-[3-(3,4,5-triméthoxybenzoyloxy)-propyl]-diazépan de formule

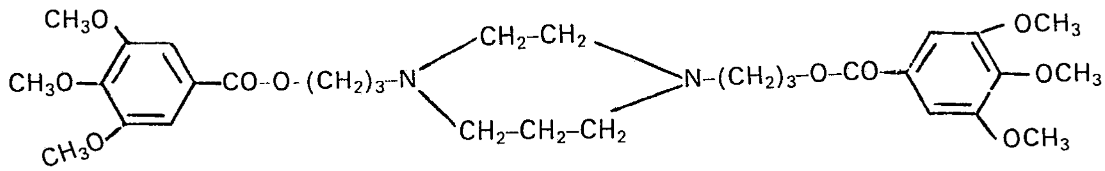

(I)

et de ses sels en une réaction en plusieurs étapes, procédé dans lequel, dans une dernière étape de la réaction, on fait réagir le 1-(3-hydroxy-propyl)-1,4-diazépan avec un 3-halogénopropanol ou avec l'alcool allylique et on introduit dans le produit de la réaction ainsi obtenue, par estérification des deux groupes hydroxy de façon connue en soi, deux radicaux 3,4,5-triméthoxybenzoyle ou on introduit dans le 1-(3-hydroxy-propyl)-1,4-diazépan, dans un ordre variable, par estérification du groupe hydroxy, un radical 3,4,5-triméthoxy-benzoyle et, sur le second atome d'azote libre du noyau diazépan, le groupe 3-[3,4,5-triméthoxy-benzoyloxy-propyle-(1) et, le cas échéant, on convertit en un sel le 1,4-bis-[3-(3,4,5-triméthoxybenzoyloxy)-propyl]-diazépan de formule I ainsi obtenu, caractérisé en ce que dans une première étape de la réaction

a) on fait tout d'abord réagir le 3-amino-propanol avec l'acrylonitrile en quantités équimolaires, sans solvant, à une température comprise entre 0 et 80 °C, puis on fait réagir le 3-(2-cyano-éthyl)-aminopropanol en solution aqueuse avec le formaldéhyde et l'acide cyanhydrique, à une température comprise entre 0 et 80 °C et à un pH de plus de 4, ou avec le formaldéhyde et un cyanure alcalin en présence d'hydrogénosulfite alcalin, pour obtenir la cyanométhyl-(2-cyano-éthyl)-(3-hydroxy-propyl)-amine, et, dans une deuxième étape de la réaction

b) on hydrogène la cyanométhyl-(2-cyano-éthyl)-(3-hydroxy-propyl)-amine obtenue, en présence d'un catalyseur d'hydrogénation et, le cas échéant, d'ammoniac, avec de l'hydrogène à une température comprise entre 20 et 180 °C et sous une pression comprise entre 1 et 300 bars, pour obtenir le 1-(3-hydroxy-propyl)-1,4-diazépan.

2. Cyanométhyl-(2-cyano-éthyl)-(3-hydroxy-propyl)-amine de formule

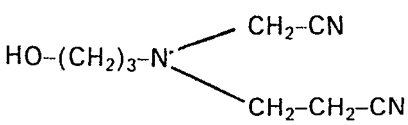

3. Procédé pour la préparation de cyanométhyl-(2-cyano-éthyl)-(3-hydroxy-propyl)-amine de formule II, caractérisé en ce qu'on fait réagir le 3-(2-cyano-éthyl)-aminopropanol en solution aqueuse avec le formaldéhyde et l'acide cyanhydrique à une température comprise entre 0 et 80 °C, à un pH de plus de 4, ou, en présence d'hydrogénosulfite alcalin, avec le formaldéhyde et un sel alcalin de l'acide cyanhydrique.

4. Procédé selon la revendication 3, caractérisé en ce qu'on utilise la (2-cyano-éthyl)-(3-hydroxy-propyl)-amine sous forme du mélange réactionnel obtenu par réaction de 3-amino-propanol et d'acrylonitrile.

5. Utilisation de cyanométhyl-(2-cyano-éthyl)-(3-hydroxy-propyl)-amine pour la préparation de 1-(3-hydroxy-propyl)-1,4-diazépan.

6. Utilisation de cyanométhyl-(2-cyano-éthyl)-(3-hydroxy-propyl)-amine selon la revendication 5, caractérisée en ce que la cyanométhyl-(2-cyano-éthyl)-(3-hydroxy-propyl)-amine est hydrogénée en présence d'un catalyseur d'hydrogénation.

7. Procédé pour la préparation de 1-(3-hydroxypropyl)-1,4-diazépan par cyclisation par voie d'hydrogénation de cyanométhyl-(2-cyano-éthyl)-(3-hydroxy-propyl)-amine en présence d'un catalyseur d'hydrogénation et éventuellement d'ammoniac, avec de l'hydrogène, à une température comprise entre 20 et 180 °C et sous une pression comprise entre 1 et 300 bars.

8. Utilisation de cyanométhyl-(2-cyano-éthyl)-(3-hydroxy-propyl)-amine pour la préparation de 1,4-bis-[3-(3,4,5-triméthoxy-benzoyloxy)-propyl]-diazépan.

## Claims

1. A process for the production of 1,4-bis-[3-(3-(3,4,5-trimethoxybenzoyloxy)-propyl]-diazepan corresponding to the following formula

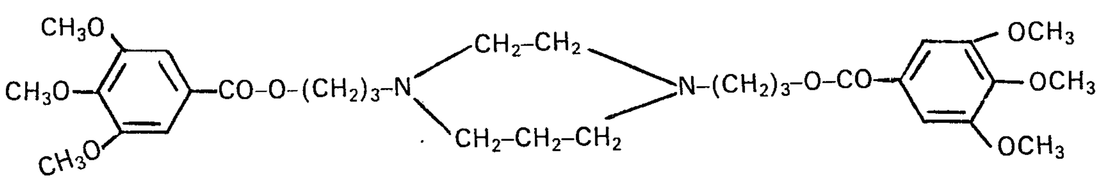

(I)

and salts thereof in a multistage reaction, 1-(3-hydroxypropyl)-1,4-diazepan being reacted with 3-halogen propanol or with allyl alcohol in a final reaction stage and two 3,4,5-trimethoxybenzoyl residues being introduced into the reaction product thus obtained by esterification of the two hydroxy groups in known manner or one 3,4,5-trimethoxybenzoyl residue and, at the second free nitrogen atom of the diazepan ring, the 3-[3,4,5-trimethoxybenzoyloxy]-1-propyl group being introduced into the 1-(3-hydroxypropyl)-1,4-diazepan in an alternating sequence by esterification of the hydroxy group and the resulting 1,4-bis- [3-(3,4,5-trimethoxybenzoyloxy)- propyl]-diazepan corresponding to formula I is optionally converted into a salt, characterized in that, in a first reaction stage

a) 3-aminopropanol is first reacted with acrylonitrile in equimolar quantities in the absence of a solvent at temperatures of 0 to 80 °C and the resulting 3-(2-cyanoethyl)-aminopropanol is subsequently reacted in aqueous solution with formaldehyde and hydrocyanic acid at temperatures of 0 to 80 °C at a pH value above 4 or with formaldehyde and an alkali cyanide in the presence of alkali hydrogen sulfite to form cyanomethyl-(2-cyanoethyl)-(3-hydroxypropyl)-amine and, in a second reaction stage

b) the cyanomethyl-(2-cyanoethyl)-(3-hydroxypropyl)-amine thus obtained is hydrogenated with hydrogen at temperatures of 20 to 180 °C and under pressures of 1 to 300 bar in the presence of a hydrogenation catalyst and, optionally, ammonia to form the 1-(3-hydroxypropyl)-1,4-diazepan.

2. Cyanomethyl-(2-cyanoethyl)-(3-hydroxypropyl)-amine corresponding to the following formula

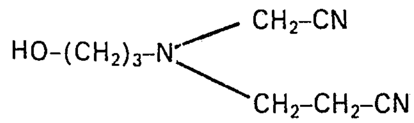

(II)

3. A process for the production of cyanomethyl-(2-cyanoethyl)- (3-hydroxypropyl)-amine corresponding to formula II, characterized in that 3-(2-cyanoethyl)-aminopropanol is reacted in aqueous solution with formaldehyde and hydrocyanic acid at temperatures of 0 to 80 °C at a pH value above 4 or with formaldehyde and an alkali salt of hydrocyanic acid in the presence of alkali hydrogen sulfite.

4. A process as claimed in claim 3, characterized in that the (2-cyanoethyl)-(3-hydroxypropyl)-amine is used in the form of the reaction mixture obtained by reaction of 3-aminopropanol and acrylonitrile.

5. The use of cyanomethyl-(2-cyanoethyl)-(3-hydroxypropyl)-amine for the production of 1-(3-hydroxypropyl)-1,4-diazepan.

6. The use of cyanomethyl-(2-cyanoethyl)-(3-hydroxypropyl)-amine as claimed in claim 5, characterized in that cyanomethyl-(2-cyanoethyl)-3-hydroxypropyl)-amine is hydrogenated in the presence of a hydrogenation catalyst.

7. A process for the production of 1-(3-hydroxypropyl)-1,4-diazepan by hydrogenating cyclization of cyanomethyl-(2-cyanoethyl)-3-hydroxypropyl)-amine with hydrogen at temperatures of 20 to 180 °C and under pressures of 1 to 300 bar in the presence of a hydrogenation catalyst and, optionally, ammonia.

8. The use of cyanomethyl-(2-cyanoethyl)-(3-hydroxypropyl)-amine for the production of 1, 4-bis-[3-(3,4,5-trimethoxybenzoyloxy)-propyl]-diazepan.